# DEMANDE DE BREVET EUROPEEN

(11) **EP 1 974 718 A1**
(43) Date de publication de la demande: **01.10.2008**
(21) Numéro de dépôt: 08151904.3
(22) Date de dépôt: 25.02.2008
(51) Int. Cl.: A61K 8/98, A61K 8/99, A61K 35/12, A61K 35/74, C12N 5/00, A61Q 17/00, A61Q 19/00

(54) **Composition cosmétique ou dermatologique comprenant un milieu de culture cellulaire**

(30) Priorité: 26.02.2007 FR 0753495
(71) Demandeur: L'Oreal, 75008 Paris (FR)
(72) Inventeur: Gueniche, Audrey, 92500, RUEIL MALMAISON (FR)
(74) Mandataire: Allab, Myriam

(57) **Abrégé**

L'invention concerne une composition cosmétique ou dermatologique contenant un milieu de culture cellulaire conditionné ou un extrait de celui-ci, ledit milieu étant susceptible d'être obtenu par contact avec au moins une culture de cellules du tractus digestif et au moins des microorganismes probiotiques. Elle concerne également l'utilisation de milieu de culture conditionné pour améliorer l'homéostasie cutanée.

## Description

La présente invention concerne des milieux de culture conditionnés et leur utilisation, notamment pour améliorer l'homéostasie de la peau ou des muqueuses. L'invention se rapporte ainsi à l'utilisation dans le domaine cosmétique ou dermatologique, mais aussi au domaine des peaux reconstruites, dont la préparation et donc les propriétés peuvent être améliorées par les milieux conditionnés, ou leurs extraits, selon l'invention.

La peau humaine est constituée de deux compartiments à savoir un compartiment superficiel, l'épiderme et un compartiment profond, le derme.
L'épiderme est composé principalement de trois types de cellules qui sont les kératinocytes (majoritaires), les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures. Le derme fournit à l'épiderme un support solide. C'est également son élément nourricier. Il est principalement constitué de fibroblastes et d'une matrice extracellulaire composée elle-même principalement de collagène, d'élastine et d'une substance fondamentale. On y trouve aussi des leucocytes, des mastocytes et des macrophages tissulaires. Enfin, Le derme est traversé par des vaisseaux sanguins et des fibres nerveuses.
La peau constitue une barrière contre les agressions extérieures, notamment : chimiques, mécaniques et infectieuses, et à ce titre un certain nombre de réactions de défense contre les facteurs environnementaux (climat, rayons ultraviolets, tabac, pollutions, infections ...) et/ou les xénobiotiques (comme par exemple certains médicaments) se produisent à son niveau.
Il est donc essentiel de préserver ou rétablir son intégrité et l'équilibre de ses différentes fonctions, notamment un équilibre entre les processus de renouvellement et de différenciation cellulaire, ou un degré d'hydratation optimum. On entend par le terme générique d'homéostasie cutanée", le maintien d'un fonctionnement et d'un équilibre physiologique de la peau saine.
Il est donc souhaitable de disposer de nouveaux moyens pour maintenir cette homéostasie cutanée, notamment pour maintenir la fonction barrière, lutter contre les signes de déshydratation ou de vieillissement de la peau.
Il est également souhaitable de disposer de milieux de culture améliorés permettant la préparation de modèles monocellulaires, pluricellulaires, en monocouche ou sous forme de tissu reconstruits, et en particulier à partir de cellules cutanées pour préparer des culture de kératinocytes, de fibroblastes, des cocultures kératinocytes/mélanocytes ou des peaux reconstruites

Ces buts et d'autres sont atteints par l'utilisation de milieux de culture dits cellulaires, ou de leurs extraits, appelés aussi milieux conditionnés.

La demande WO 02/098365 (Advanced Tissue Science) propose l'utilisation de milieux de culture conditionnés pour des applications cosmétiques ou pharmaceutiques. Les milieux conditionnés sont obtenus par culture de cellules de peau humaine, en particulier de fibroblastes ou kératinocytes. Ces cellules sont génétiquement modifiées pour augmenter leur production de facteurs de croissance ou d'antioxydants dans le milieu, qui contient généralement un collagène soluble.
US 2005/0249691 décrit des compositions cosmétiques ou dermatologiques comprenant un milieu de culture pour des cellules de la peau ou des couches cornées, en association avec une matrice gélifiée ; les compositions contiennent obligatoirement du collagène, des chitosanes et des glycosaminoglycanes.
US 2006/0182701 décrit également des compositions cosmétiques ou dermatologiques comprenant un milieu de culture de cellules de la peau. Il vise à fournir aux cellules de la peau sur lesquelles elles sont appliquées un milieu qui permettra leur développement analogue à ce qui est obtenu in vitro.

De manière inattendue, il a maintenant été trouvé dans le cadre de la présente invention qu'un milieu de culture conditionné par des cellules complètement différentes des cellules cutanées, peut être utilisé pour favoriser le bon état de la peau ou de ses annexes.
C'est pourquoi la présente invention a pour objet une composition cosmétique ou dermatologique contenant un milieu de culture cellulaire ou un extrait de celui-ci, ledit milieu étant susceptible d'être obtenu par contact avec au moins une culture de cellules du tractus digestif et au moins des microorganismes probiotiques.
Avantageusement, le milieu de culture cellulaire, aussi appelé milieu conditionné selon la présente invention, est susceptible d'être obtenu par contact avec des cellules du sang périphérique, ou des cellules dérivés de cellules de sang périphérique. De telles cellules sont en particulier des leucocytes, mais pourront également être choisies parmi des lignées cellulaires immortalisées, dérivées de lignées de cellules sanguines, notamment de monocytes; de manière non limitative on peut citer des cellules commercialisées par la société LGC Promochem, sous les désignations suivantes:
SC (code ATCC : CRL-9855)
AML-193 (code ATCC : CRL-9589)
THP-1 (code ATCC : TIB-202).
Ces cellules de sang périphériques ou leurs dérivées, notamment les leucocytes peuvent par exemple être en culture dans le milieu avec les cellules de tractus digestif.

De préférence, ces leucocytes comprennent majoritairement des lymphocytes, mais peuvent aussi comporter d'autres cellules de sang périphérique telles que les monocytes, qui sont mises en contact avec le milieu.
Selon l'un des modes de réalisation avantageux de l'invention, la culture de cellules du tractus digestif est une culture tridimensionnelle.
Les cellules du tractus digestif utiles pour la mise en oeuvre de l'invention pourront être dérivées de différentes organes parties du tractus digestif, tel que l'oesophage, l'estomac ou l'intestin. Avantageusement on utilise des cellules dérivées de l'épithélium intestinal; de telles cellules épithéliales de l'intestin sont connues de l'homme du métier. On peut citer, à titre d'exemple non limitatif, les cellules humaines d'origine intestinale connues sous l'appellation CaCO-2, HT29 ou T84.
Les souches correspondantes sont déposées dans les collections de culture de l'ATCC avec les n ° suivants:
Caco 2: ATCC N ° HTB-37
HT29: ATCC N ° HTB-38
T84: ATCC N ° CCL-248

Au sens de la présente invention, on entend par "microorganisme probiotique", un microorganisme vivant qui, lorsqu'il est consommé en quantité adéquate, a un effet positif sur la santé de son hôte "joint FAO/WHO Expert Consultation on Evaluation of Health and Nutritional Properties of Probiotic in Food Including Powder Milk with Live Lactic Acid Bacteria, 6 octobre 2001 ", et qui peut en particulier améliorer l'équilibre microbien intestinal.
Les probiotiques peuvent être introduit dans le milieu de culture cellulaire sous forme d'une suspension de cellules vivantes, dont la concentration sera adaptée par l'homme du métier en fonction de la quantité des autres constituants du mélange. A titre indicatif, on peut utiliser un inoculum comprenant environ 10⁴ à 10⁹ufc/ml (ufc signifiant "unité formant une colonie" ou cfu signifiant "colony forming unit" c'est-à-dire "unité capable de former une colonie"), de préférence au moins 10⁵ cfu/ml. L'inoculum de probiotiques peut de façon classique être à une concentration d'environ 10⁶ à 10⁷ cfu.

Les microorganismes convenant à l'invention peuvent être choisis notamment parmi les ascomycetes telles que *Saccharomyces, Yarrowia, Kluyveromyces, Torulaspora, Schizosaccharomyces pombe, Debaromyces, Candida, Pichia, Aspergillus* et *Penicillium,* des bactéries du genre *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus, Lactobacillus* et leurs mélanges.
Comme ascomycetes convenant tout particulièrement à la présente invention, on peut en particulier citer *Yarrowia lipolitica et Kluyveromyces lactis,* de même que *Torulaspora, Schizosaccharamyces pombe, Candida* et *Pichia* ou encore des levures comme *Saccharomyces cerevisiae* ou *boulardii.*
En ce qui concerne les microorganismes probiotiques, ce sont les genres bactériens et de levure suivants qui sont généralement utilisés :
- les bactéries lactiques : qui produisent par fermentation du sucre de l'acide lactique. Suivant leur morphologies ont les divisent en deux groupes :
   - F; *Lacobacillus species : Lactobacillus acidophilus (LC1, NCFB 1748) ; amylovorus, casei (Shirota), rhamnosus* (souche GG), *brevis, crispatus, delbrueckii (subsp bulgaricus, lactis), fermentum, helveticus, gallinarum, gasseri johnsonii, paracasei, plantarum, reuteri, rhamnosus, salivarius), alimentarius, curvatus, casei subsp. casei, sake*
   - *Gocci : Enterococcus (faecalis, faecium), Lactococcus lactis (subspp lactis ou cremoris), Leuconstoc mesenteroides* subsp *dextranicum, Pediococcus acidilactici, Sporolactobacillus inulinus, Streptococcus salvarius subsp. Thermophilus, Streptococcus thermophilus, Staphylococccus carnosus, Staphylococcus xylosus*
- Les bifidobactéries ou *Bifidobacterium species : Bifidobacterium adolescentis, animalis, bifidum, breve, lactis, longum, infantis, pseudocatenulatum*
- Les autres bactéries sporulées : *Bacillus (cereus var toyo ou subtilis), Bacillus coagulans, Bacillus licheniformis, Escherichia coli strain nissle, Propionibacterium freudenreichii,*
Des exemples spécifiques de microorganismes probiotiques sont *Bifidobacterium adolescentis, Bifidobacterium animalis, Bifidobacterium bifidum, Bifidobacterium breve, Bifidobacterium lactis, Bifidobacterium longum, Bifidobacterium infantis, Bifidobacterium pseudocatenulatum, Lactobacillus acidophilus (LC1, NCFB 1748) ; Lactobacillus amylovorus, Lactobacillus casei (Shirota), Lactobacillus rhamnosus* (souche GG), *Lactobacillus brevis, Lactobacillus crispatus, Lactobacillus delbrueckii (subsp bulgaricus, lactis), Lactobacillus fermentum, Lactobacillus helveticus, Lactobacillus gallinarum, Lactobacillus gasseri, Lactobacillus johnsonii, Lactobacillus paracasei, Lactobacillus plantarum, Lactobacillus reuteri, Lactobacillus rhamnosus, Lactobacillus salivarius), Lactobacillus alimentarius, Lactobacillus curvatus, Lactobacillus casei subsp. casei, Lactobacillus sake Lactococcus lactis, Enterococcus (faecalis, faecium), Lactococcus lactis (subspp lactis ou cremoris), Leuconstoc mesenteroides* subsp *dextranicum, Pediococcus acidilactici, Sporolactobacillus inulinus, Streptococcus salvarius subsp. Thermophilus, Streptococcus thermophilus, Staphylococccus carnosus, Staphylococcus xylosus, Saccharomyces (cerevisiae* ou encore *boulardii), Bacillus (cereus var toyo ou subtilis), Bacillus coagulans, Bacillus licheniformis, Escherichia coli strain nissle, Propionibacterium freudenreichii* et leurs mélanges.
Avantageusement, au moins un microorganisme probiotique est choisi parmi les bactéries lactiques, les bifidobactéries et les levures Saccharomyces.
Plus particulièrement, il s'agit de microorganismes probiotiques issus du groupe des bactéries lactiques, comme notamment les *Lactobacillus* et/ou les *Bifidobacterium,* en particulier les *Lactobacillus.* A titre illustratif de ces bactéries lactiques, on peut plus particulièrement citer les *Lactobacillus johnsonii, Lactobacillus reuteri, Lactobacillus rhamnosus, Lactobacillus paracasei, Lactobacillus casei* ou *Bifidobacterium bifidum, Bifidobacterium breve, Bifidobacterium longum, Bifidobacterium animalis, Bifidobacterium lactis, Bifidobacterium infantis, Bifidobacterium adolescentis* ou *Bifidobacterium pseudocatenulatum* et leurs mélanges.
Les espèces convenant tout particulièrement sont les *Lactobacillus johnsonii, Lactobacillus paracasei, Bifidobacterium adolescentis, Bifidobacterium longum* et *Bifidobacterum Lactis NCC 2818 (encore désigné Bb12 ATCC 27536) ;* on peut citer en particulier les souches suivantes, déposées suivant le traité de Budapest à la collection de culture de l'Institut Pasteur (28 rue du Docteur Roux, F-75024 Paris cedex 15) les 30/06/92, 12/01 /99, 15/04/99, 15/04/99, 07/06/05: *Lactobacillus johnsonii* (CNCM 1-1225), *Lactobacillus paracasei* (CNCM 1-2116), *Bifidobacterium adolescentis* (CNCM 1-2168), *Bifidobacterium longum* (CNCM 1-2170) et *Bifidobacterum lactis* (CNCM I-3446), et le genre *Bifidobacterium longum (BB536).* La souche de *Bifidobacterium lactis* CNCM 1-3446 peut être obtenue chez Hansen (Chr. Hansen A/S, 10-12 Boege Alle, P.O. Box 407, DK-2970 Hoersholm, Danemark).

Le milieu de culture cellulaire avec lequel les cellules sont mises en contact sera tout milieu nutritif convenant à la survie et/ou la culture des différents types cellulaires mis en oeuvre. Il contient généralement une source de carbone et d'azote, des minéraux, des vitamines et/ou des oligoélements, comme par exemple des acides aminés, des sucres, des protéines, des acides gras.
Les cellules peuvent être cultivées en dehors de leur tissue d'origine. Pour cela elles doivent être cultivés dans un environnement proches de leur condition naturelle dans le tissu. Ces cultures requièrent des facteurs vitaux dans leur milieu de culture. Cet environnement doit avoir une composition définie composée de minéraux et de biomatériaux connu sous le nom de milieu de culture. Ces milieux de culture sont en général fournis par des fournisseurs spécialistes et possèdent des caractéristiques particulières en fonction des types cellulaires. Les milieux de culture contiennent généralement en plus de l'eau, une source de carbone et d'azote, des phosphates et des sulfates, des minéraux et des facteurs de croissances et des vitamines en quantité adéquate.
Les cellules cultivées dans ce type de milieu nécessitent souvent l'ajout de sérum. Ce sérum a une composition complexe et apporte aux cellules au moins des hormones, des facteurs d'adhésion et des acides aminés. Ce sérum pourra par exemple être remplacé en tout ou partie par l'ajout des milieux conditionnés de l'invention. Ces milieux conditionnés pourront aussi être ajouté en plus dans le milieu de culture comme enrichissement.

L'homme du métier déterminera aisément des milieux adaptés. On peut citer, de manière non limitative, le milieu RPMI ou le milieu DMEM (Dulbecco modified Eagle medium) le Dulbecco's Modified Eagle's Medium (DMEM), le Minimal Essential Medium (MEM), le M199, le RPMI 1640 ou l'Iscove's Modified Dulbecco's Medium (EDMEM), le Ham's F-12, le Ham's F-1 0, le NCTC 109 et le NCTC 135.

Ces milieux peuvent être complémentés par tout additif classiquement utilisé en culture cellulaire tel que, par exemple et de manière non limitative, des précurseurs de phospholipides, des acides aminés non essentiels, des acides nucléiques, des vitamines, des antibiotiques, des co-facteurs enzymatiques, des sels minéraux, de l'insuline, de la transferrine, de la triiodothyronine, de l'éthanolamine, de l'o-phosphoryl-éthanolamine ou des facteurs de croissance tels que le facteur de croissance nerveuse ou la neurotrophine-3.

Les concentrations des différents additifs usuellement utilisés pour complémenter les milieux de culture cellulaire peuvent être déterminés et adaptés par l'homme de l'art, notamment selon le type de cellules à cultiver.

D'autres milieux sont décrits dans HAM and McKEEHAN, « Methods in Enzymology », 58:44-93, 1979, ou encore dans BOTTENSTEIN et al., « Methods in Enzymology », 58:94-109, 1979, dont le contenu est incorporé ici par référence.

Par ailleurs, il est également possible d'utiliser des mélanges de différents milieux notamment des milieux précités, tels que par exemple un mélange de DMEM/HAM F12.
Ces milieux peuvent être supplémentés en facteurs de croissance spécifique, ou par exemple avec du sérum, mais ce dernier composant peut aussi être absent.
Selon un mode de réalisation, on n'ajoute pas de collagène dans le milieu de culture.
Ce milieu de culture peut être liquide, semi-liquide, gélifié ou solide, de préférence au moins partiellement liquide.

Par extrait du milieu de culture cellulaire conditionné on entend notamment toute fraction ou sous composé de ces milieux conditionnés obtenus par dialyse, fractionnement, séparation de phase, chromatographie par filtration, chromatographie par affinité, précipitation, concentration, lyophilisation....
Les milieux conditionnés peuvent être générés à partir de milieux qui peuvent être dépourvus de sérum et de produit animal. Ce milieu conditionné est de préférence obtenu après stimulation des cellules du tractus digestif en présence de leucocytes humains, par des probiotiques et en particulier des probiotiques de l'espèce des lactobacilles ou des bifidobactéries.

Avantageusement, le milieu de culture (ou ses extraits) utilisé dans les compositions de l'invention est un milieu stabilisé, c'est-à-dire qu'il a subi une manipulation destinée à le préserver dans l'état dans lequel il se trouvait à un instant donné choisi, généralement à la fin de son processus de préparation, tout en ayant conservé ses propriétés intrinsèques. En particulier, cette manipulation est destinée à rendre ledit milieu stérile, c'est-à-dire incapable de permettre la croissance de microorganismes tout en préservant les propriétés biologiques dont il est doté. La stabilisation du milieu de culture peut être obtenue par toute technique connue de l'homme du métier, comme par exemple la filtration stérilisante, l'autoclavage, l'ultra haute température (technique UHT), la stérilisation haute pression, les rayonnements y ou la congélation.
Les milieux de culture conditionnés selon l'invention contiennent généralement de l'IL-10, qui n'était pas présent dans les différents constituants du milieu d'origine. Les quantités d'IL-10 pourront varier selon les conditions de préparation du milieu conditionné de l'invention, mais correspondront en général à une concentration supérieure ou égale à 20 pg/ml de milieu récupéré après contact avec les différents types cellulaires.

De manière inattendue, il a maintenant été trouvé que de tels milieux conditionnés présentent des propriétés avantageuses pour la peau et/ou les phanères.
C'est pourquoi la présente invention a également pour objet l'utilisation cosmétique d'au moins un milieu de culture cellulaire conditionné susceptible d'être obtenu par contact avec au moins une culture de cellules du tractus digestif et au moins des microorganismes probiotiques, tels que définis précédemment, ou d'un extrait dudit milieu de culture cellulaire comme agent pour améliorer l'homéostasie de la peau et/ou des phanères.
Avantageusement, le milieu conditionné sera obtenu par contact avec en outre des cellules de sang périphérique ou leurs dérivées, notamment des leucocytes.

Dans la présente description, à moins qu'il n'en soit spécifié différemment, on entend par peau l'ensemble du revêtement du corps humain, c'est-à-dire la peau, les muqueuses et le cuir chevelu.
Par phanères on entend les ongles, les cheveux et les poils, tels que les cils.

En particulier, l'invention concerne l'utilisation d'un milieu de culture ou de l'un de ses extraits comme agent pour améliorer la fonction barrière de la peau ou du cuir chevelu.
Selon un autre aspect de l'invention, le milieu de culture ou ses extraits est utile comme agent pour améliorer l'hydratation de la peau ou du cuir chevelu.
Ils seront ainsi particulièrement utiles pour lutter contre les signes des peaux sèches, notamment des peaux sèches et sensibles.

D'une manière générale, les peaux sensibles se définissent par une réactivité particulière de la peau.
Cette réactivité cutanée se traduit classiquement par la manifestation de signes d'inconfort en réponse à la mise en contact du sujet avec un élément déclenchant qui peut avoir diverses origines. Il peut s'agir de l'application d'un produit cosmétique en surface de la peau sensible, de la prise d'aliments, de l'exposition à des variations brutales de températures, à la pollution atmosphérique et/ou à des rayons aux ultraviolets ou infrarouges. Il existe également des facteurs associés comme l'âge et le type de peau. Ainsi les peaux sensibles sont plus fréquentes parmi les peaux sèches ou grasses selon les critères cosmétiques de classification des types de peau. Les sujets à peau sensibles peuvent ainsi être des sujets à peau irritable ou réactive, ou à peau intolérante.
L'apparition de ces signes d'inconfort, qui apparaissent dans les minutes qui suivent la mise en contact du sujet avec l'élément déclenchant, est une des caractéristiques essentielles des peaux sensibles. Il s'agit principalement de sensations dysesthésiques. On entend par sensations dysesthésiques, des sensations ressenties dans une zone cutanée comme les picotements, fourmillements, démangeaisons, échauffements, inconforts, tiraillements, etc. Ces signes subjectifs existent le plus souvent en l'absence de signes chimiques visibles tels que la rougeur et les desquamations, et dans tous les cas sans qu'il y ait oedème. On sait aujourd'hui que ces réactions d'irritation et d'intolérance cutanée sont notamment liées à une libération de neuropeptides par les terminaisons nerveuses de l'épiderme et du derme.
Lors des manifestations aigües de sensibilité cutanée, il peut y avoir déclenchement d'une réaction neurogène. Ses conséquences à long terme peuvent contribuer à entretenir les phénomènes inflammatoires ou hyperalgiques, et donc à mener à la chronicité du processus.

Par opposition aux peaux qualifiées d'allergiques, la réactivité d'une peau sensible ne relève pas d'un processus immunologique c'est-à-dire ne se produit pas uniquement au niveau d'une peau déjà sensibilisée, en réponse à la présence d'un allergène. Son mécanisme de réponse est dit "aspécifique". Elle est, à ce titre, à distinguer des peaux manifestant des réactions inflammatoires et allergiques de type dermatose, eczéma, et/ou ichtyoses.

Dans le cas de l'invention les milieux conditionnés permettent la prévention et/ou le traitement des peaux qualifiées de sensibles, en particulier dans le cas où cette peau sensible est associée à une peau sèche. La peau sèche se manifeste essentiellement par une sensation de tiraillement et/ou de tension. Elle est souvent associée à une baisse du taux d'hydratation cutanée et à une altération de la fonction barrière, mesurée par la perte insensible en eau.

Comme précisé précédemment, une peau sensible est différente d'une peau allergique. Sa réactivité ne relève pas d'un processus immunologique et se traduit généralement uniquement par des sensations dysesthésiques, éventuellement associées à une rougeur mais sans gonflement ni oedème.
Les "peaux sensibles" peuvent notamment être caractérisées par des outils d'évaluation de la réaction sensorielle de la peau. Les premiers outils se sont inspirés dès leur conception de la caractéristique essentielle des peaux sensibles à savoir la présence de signes d'inconfort induits par une application topique. Ainsi, le "stinging test" à l'acide lactique a été le premier test proposé. Il est réalisé par relevé des sensations de picotements rapportées par un volontaire après application d'une solution d'acide lactique à 10 % sur les ailes du nez. Les sujets rapportant des sensations modérées ou fortes de picotements sont appelées "stingers" et considérés comme étant à peau sensible. En raison de cette sensibilité cutanée à l'application topique de produit, ces sujets sont alors sélectionnés pour tester des produits dits peaux sensibles. Plus récemment, pour activer spécifiquement les terminaisons nerveuses périphériques, impliquées dans l'inconfort et appelées nocicepteurs, récemment identifiées comme étant impliquées dans la peau sensible, de nouveaux tests ont été proposés qui utilisent précisément d'autres inducteurs d'inconfort comme la capsaïcine, notamment dans la demande EP 1 374 913.
Au sens de la présente invention, les peaux sensibles couvrent les peaux irritables et les peaux intolérantes.
Une peau intolérante est une peau qui réagit par des sensations d'échauffement, de tiraillements, de fourmillements et/ou de rougeurs, à différents facteurs tels que l'application de produits cosmétiques ou dermatologiques ou de savon.
Une peau irritable est une peau qui réagit par des démangeaisons ou par des picotements, à différents facteurs tels que l'environnement, les émotions, les aliments, le vent, les frottements, le rasoir, l'eau dure à forte concentration de calcaire, les variations de température, 1' humidité ou la laine.

Ces deux types de peau sont généralement associés à une sécheresse cutanée avec ou sans dartres ou à une peau qui présente un érythème. Les peaux intolérantes peuvent cependant aussi être associées à une peau acnéique, voire même rosacéiforme, avec ou sans dartres.

Comme précisé précédemment, la sécheresse cutanée est souvent associée à une baisse du taux d'hydratation cutanée, évalué par cornéométrie, ainsi qu'à une altération de la fonction barrière, mesurée par la perte insensible en eau.
La peau sèche se manifeste pour l'essentiel par une sensation de tiraillements et/ou de tension. Celle-ci est aussi rugueuse au toucher et apparaît couverte de squames. Lorsque la peau est légèrement sèche, ces squames sont abondantes mais peu visibles à l'oeil nu.
L'origine de cette sécheresse cutanée peut être de type constitutionnel ou acquis.
Dans le cas de peau sèche acquise, l'intervention de paramètres extérieurs tels que l'exposition aux agents chimiques, à des conditions climatiques difficiles, aux rayons solaires ou bien encore certains traitements thérapeutiques (rétinoïdes, par exemple) est déterminante. Sous ces influences extérieures, la peau peut devenir alors momentanément et localement sèche. Cela peut concerner tout type de peau.

Dans le cas des peaux sèches constitutionnelles non pathologiques, la sévérité de l'état de sécheresse peut dépendre des facteurs extérieurs déjà évoqués. Rentrent dans cette catégorie de peau, la peau sénile (caractérisée par une diminution générale du métabolisme cutané avec l'âge), la peau fragile (très sensible aux facteurs extérieurs et souvent accompagnée d'érythème et de rosacée) et la xérose vulgaire (d'origine génétique probable et se manifestant en priorité sur le visage, les membres et le dos des mains). Il doit être entendu que l'état de peau sèche est un état physiologique qui est indépendant de toute manifestation pathologique et correspond à une peau saine dont on souhaite améliorer le confort et l'aspect par des moyens cosmétiques.
Les compositions, procédés et utilisations selon l'invention, s'avèrent ainsi tout particulièrement efficaces pour prévenir et/ou traiter les peaux sensibles et/ou sèches et plus particulièrement les peaux dites réactives, irritables et/ou intolérantes, les peaux sèches acquises et/ou les peaux sèches constitutionnelles.

L'invention a également pour objet l'utilisation d'un milieu conditionné ou de l'un de ses extraits pour lutter contre les signes du vieillissement, qu'il soit chronologique ou photo-induit.
Au cours du temps, il apparaît différents signes sur la peau très caractéristiques du vieillissement intrinsèque, se traduisant notamment par un modification de la structure et des fonctions cutanées. L'autre composante est exogène (Yaar et Gilchrest, J Invest Dermatol, 1998). En effet, le vieillissement peut-être accéléré par des facteurs environnementaux tels qu'une exposition répétée de la peau à la lumière solaire et notamment aux rayonnements ultraviolets A et B, à la pollution, notamment aux particules de diesel et à la fumée de cigarette.
La toxicité des polluants atmosphériques, notamment des polluants gazeux tels que le dioxyde de soufre, l'ozone et les oxydes d'azote sur les constituants de la peau (fibres, cellules, enzymes) est liée notamment à leur activité d'initiateurs de radicaux libres, sources de phénomènes d'oxydation qui provoquent chez les êtres vivants des dommages cellulaires. Ces dommages induisent un vieillissement accéléré de la peau.
Les signes du vieillissement cutané sont notamment les rides et ridules, la peau molle ou la peau amincie.
L'un des objets de l'invention est l'utilisation d'un milieu conditionné ou de l'un de ses extraits dans ou pour la préparation d'une composition, en particulier d'une composition cosmétique, le milieu conditionné, son extrait et/ou la composition étant destiné à améliorer l'homéostasie cutané et/ou la fonction barrière et /ou l'hydratation de la peau, du cuir chevelu et/ou des phanères.

L'invention a également pour objet procédé cosmétique pour améliorer l'homéostasie cutané et/ou la fonction barrière et /ou l'hydratation de la peau ou du cuir chevelu, caractérisé en ce qu'on applique sur la peau ou le cuir chevelu au moins un milieu de culture cellulaire ou au moins un extrait de milieu de culture, ou encore une composition les contenant, ledit milieu étant susceptible d'être obtenu par contact avec au moins une culture de cellules du tractus digestif et au moins des microorganismes probiotiques, tels que définis dans ce qui précède. Le milieu de culture cellulaire ou l'extrait du milieu de culture sont particulièrement utiles comme agent pour améliorer l'homéostasie cutanée.

Selon un autre de ses aspects, l'invention concerne l'utilisation d'au moins un milieu de culture cellulaire ou au moins un extrait de milieu de culture, ledit milieu étant susceptible d'être obtenu par contact avec au moins une culture de cellules du tractus digestif et au moins des microorganismes probiotiques, tels que définis dans ce qui précède, pour la préparation d'une composition destinée au traitement et/ou à la prévention des signes du stress cutané. Une telle composition peut être une composition à usage cosmétique ou pharmaceutique, notamment une composition dermatologique.

La quantité de milieu de culture conditionné ou de ses extraits selon l'invention dans les compositions sera adapté par l'homme du métier pour obtenir l'effet recherché. La quantité efficace sera ainsi déterminée par des techniques de routine, comprenant des essais in vitro et des dosages in vivo, et dépendra notamment du type d'extrait utilisé et du type de formulation retenue.
A titre indicatif, la concentration de matière active de milieu conditionné pourra être comprise entre 0,001 et 50 % en poids par rapport au poids total de la composition, notamment inférieure ou égale à 10%, mais ces quantités pourront varier sans inconvénient.

Les compositions selon l'invention contiennent également un milieu physiologiquement acceptable, en particulier un milieu cosmétiquement ou pharmaceutiquement acceptable.

Par composition ou produit cosmétique au sens de l'invention on entend notamment toute substance ou préparation destinée à être mise en contact avec les diverses parties superficielles du corps humain (épiderme, système pileux et capillaire, ongles, lèvres et organes génitaux externes) ou avec les dents et les muqueuses buccales en vue, exclusivement ou principalement, de les nettoyer, de les parfumer, d'en modifier l'aspect et/ou de corriger les odeurs corporelles et/ou de les protéger ou de les maintenir en bon état (directive cosmétique 76/768/CEE modifiée).
Ces compositions ont généralement une odeur et un aspect rendant agréable leur application sur le corps humain.

De préférence, une composition de l'invention est appliquée sur la peau ou les muqueuses.
Selon le mode d'administration considéré, elle peut se présenter sous toutes les formes galéniques normalement utilisées.
Pour une application topique sur la peau ou les muqueuses, la composition peut avoir la forme notamment de solutions aqueuses ou huileuses ou de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique ou de mousses. Elles peuvent également se présenter sous forme de microsphères ou nanosphères ou de vésicules lipidiques ou polymériques ou de patchs polymériques et d'hydrogels permettant une libération contrôlée.
Selon un mode de réalisation avantageux, la composition est une composition dermocosmétique contenant, dans un support cosmétiquement ou pharmaceutiquement acceptable, au moins un un milieu conditionné ou un extrait selon l'invention à raison d'au moins 0,001 % en poids par rapport au poids total de la composition, et de préférence de 0,05 à 3%.
Ces compositions sont préparées selon les méthodes usuelles.

Les quantités des différents constituants des compositions selon l'invention sont celles classiquement utilisées dans les domaines considérés. Les constituants et leurs quantités seront de préférence choisis de manière à ne pas interagir, en la diminuant, avec l'activité du milieu conditionné ou de ses extraits.
Dans le domaine de la cosmétique, ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes de démaquillage, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions de bronzage artificiel, des compositions pour le bain, des compositions désodorisantes comprenant un agent bactéricide, des gels ou lotions après-rasage.
Les compositions selon l'invention peuvent également consister en des préparations solides constituant des savons ou des pains de nettoyage.
Les compositions peuvent aussi être conditionnées sous forme de composition pour aérosol comprenant également un agent propulseur sous pression.

Une composition selon l'invention peut aussi être une composition pour les soins du cuir chevelu, et notamment un shampoing, une lotion de mise en plis, une lotion traitante, une crème ou un gel coiffant, de lotions restructurantes pour les cheveux, une lotion ou un gel antichute, un shampoing antiparasitaire, antipelliculaire etc.
Une composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut varier d'environ 5 % à 80 % en poids, et de préférence d'environ 5 % à 50 % en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 % à 30 % en poids, et de préférence de 0,5 % à 20 % en poids par rapport au poids total de la composition. L'émulsion peut, en outre, contenir des vésicules lipidiques.
Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeurs et les matières colorantes. Les quantités de ces différents adjuvants sont celles classiquement utilisées dans le domaine cosmétique, et par exemple varient d'environ 0,01 % à 10 % du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique). Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{®} 63 par la société Gattefosse.

Comme solvants utilisables dans l'invention, on peut citer les alcools inférieurs notamment l'éthanol et l'isopropanol et le propylène glycol.
Comme gélifiants hydrophiles utilisables dans l'invention, on peut citer les polymères carboxyvinyliques (carbomer^{®}), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, l'éthylcellulose et le polyéthylène.

Dans les compositions dermocosmétiques selon l'invention, l'extrait de milieu conditionné peut être combiné avec des rétinoïdes ou des corticostéroïdes, ou associé avec des anti-radicaux libres, avec des alpha-hydroxy ou alpha-céto acides ou leurs dérivés, ou encore des bloqueurs de canaux ioniques.
Les compositions dermocosmétiques selon l'invention peuvent, en outre, contenir des additifs inertes ou même pharmacodynamiquement ou cosmétiquement actifs ou des combinaisons des ces additifs et notamment : des agents mouillants, des agents dépigmentant tels que l'hydroquinone, l'acide azelaïque, l'acide caféïque ou l'acide kojique ; des émollients ; des agents hydratants comme le glycérol, le PEG-400, l'urée ; des agents anti-âge, des agents antiséborrhéiques ou antiacnéiques, tels que la S-carboxyméthylcystéine, la S-benzyl-cystéamine, leurs sels et leurs dérivés, ou le peroxyle de benzoyle ; des antibiotiques comme l'érythromycine et ses esters, la néomycine, la clindamycine et ses esters, les tétracyclines ; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-methyl 1,2,4-benzothiadiazine 1,1-dioxyde) et le Phénytoïne (5,4-diphényl-imidazoline 2,4-dione) ; des agents anti-inflammatoires non stéroïdiens, des carotenoïdes et notamment le β-carotène ; des agents anti-psoriasiques tels que l'anthraline et ses dérivés et enfin, les eicosa-5,8,11,14-tétraéno'ique et eicosa-5,8,11-trynoïque, leurs esters et les amides.
Les agents anti-âge sont notamment des agents anti-glycation, inhibiteurs de NO-synthase, ou stimulant la synthèse de macromolécules dermiques ou épidermiques ou empêchant leur dégradation, ou stimulant la prolifération des fibroblastes ou des kératinocytes et/ou la différenciation des kératinocytes.

Par "agent hydratant", on entend :
- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-a-benzoyl-L-arginine ;
- soit un composé activant les glandes sébacées tel que les dérivés stéroïdiens (dont la DHEA) et la vitamine D et ses dérivés.

Ces composés peuvent représenter de 0,001% à 30%, et de préférence de 0,01 à 20%, du poids total de la composition selon l'invention.

Des exemples d'inhibiteurs de NO-synthase convenant à une utilisation dans la présente invention comprennent notamment un extrait de végétal de l'espèce *Vitis vinifera* qui est notamment commercialisé par la société Euromed sous la dénomination Leucocyanidines de raisins extra, ou encore par la société Indena sous la dénomination Leucoselect^{®}, ou enfin par la société Hansen sous la dénomination Extrait de marc de raisin ; un extrait de végétal de l'espèce *Olea europaea* qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce *Gingko biloba* qui est de préférence un extrait aqueux sec de ce végétal vendu par la société Beaufour sous le nom commercial Ginkgo biloba extrait standard.

Parmi les actifs stimulant les macromolécules du derme ou empêchant leur dégradation, on peut citer ceux qui agissent :
- soit sur la synthèse du collagène, tels que les extraits de Centella asiatica ; les asiaticosides et dérivés ; l'acide ascorbique ou vitamine C et ses dérivés, tels que l'ascorbyl glucoside (vendu par la société Hayashibara ; les peptides de synthèse tels que la iamin, le palmitoyle de tripeptide glycine-histidine-lysine vendu sous la dénomination « Biopeptide CL » par la société SEDERMA ; les peptides extraits de végétaux, tels que l'hydrolysat de soja commercialisé par la société COLETICA sous la dénomination commerciale Phytokine^{®} ; les extraits de fibres de soja, tel que celui vendu sous la dénomination « Raffermine » par la société SILAB ; les hormones végétales telles que les auxines et les lignanes ; le palmitoyle de pentapeptide lysine-thréonine-thréonine-lysine-sérine vendu notamment sous la dénomination « MATRIXYL » par la société SEDERMA ; le diméthyl amino éthanol ; les extraits de rizhome de Bupleurum Chinensis, tels que ceux vendus sous les dénominations « PLEURIMINCYL », « LIPOCARE » par la société SEDERMA ; les hydrolysats de protéine de blé acylés notamment par un groupement palmitoyle, tel que celui vendu sous la dénomination « LIPACID PVB » par la société SEPPIC ; la créatine ; le coenzyme Q10 ; le rétinol, le dipalmitoyl hydroxyproline, notamment commercialisé par la société SEPPIC sous la dénomination « SEPILIFT DPHP », les extraits de trèfle rouge *(trifolium pratense)* contenant des isoflavones ;
- soit sur la synthèse d'élastine, tels que l'extrait de Saccharomyces Cerivisiae commercialisé par la société LSN sous la dénomination commerciale Cytovitin^{®} ; et l'extrait d'algue Macrocystis pyrifera commercialisé par la société SECMA sous la dénomination commerciale Kelpadelie^{®} ;
- soit sur la synthèse des glycosaminoglycanes, tels que le produit de fermentation du lait par lactobacillus vulgaris, commercialisé par la société BROOKS sous la dénomination commerciale Biomin yogourth^{®} ; l'extrait d'algue brune Padina pavonica commercialisé par la société ALBAN MÜLLER sous la dénomination commerciale HSP3^{®} ; et l'extrait de Saccharomyces cerevisiae disponible notamment auprès de la société SILAB sous la dénomination commerciale Firmalift^{®} ou auprès de la société LSN sous la dénomination commerciale Cytovitin^{®} ;
- soit sur la synthèse de la fibronectine, tels que l'extrait de zooplancton Salina commercialisé par la société SEPORGA sous la dénomination commerciale GP4G^{®} ; l'extrait de levure disponible notamment auprès de la société ALBAN MÜLLER sous la dénomination commerciale Drieline^{®} ; et le palmitoyl pentapeptide commercialisé par la société SEDERMA sous la dénomination commerciale Matrixil^{®} ;
- soit sur la synthèse de composés présents au niveau de la jonction dermo-épidermique (tels que les collagènes VII et/ou IV) et /ou la laminine, tels que le dipalmitoyl hydroxyproline, notamment commercialisé par la société SEPPIC sous la dénomination « SEPPILIFT DPHP », le sulfate de phytostérol , tel que celui commercialisé par la société VINCIENCE sous la dénomination « PHYTOCOHESINE» ;
- soit sur l'inhibition des métalloprotéinases (métalloprotéinases matricielles ou MMP) telles que plus particulièrement les MMP 1, 2, 3, 9. On peut citer : les rétinoïdes et dérivés, les oligopeptides et les lipopeptides, les lipoaminoacides, l'extrait de malt commercialisé par la société COLETICA sous la dénomination commerciale Collalift^{®} ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB^{®}), de trèfle rouge (commercialisé par exemple par la société SEDERMA sous la dénomination « STEROCARE^{®}» ), de lin, de kakkon ou de sauge ; les extraits de cucurma longa ; les extraits de Siegesbeckia (commercialisé par exemple par la société Sederma) ;
- soit sur l'inhibition des sérine protéases telles que l'élastase leucocytaire ou la cathepsine G. On peut citer : l'extrait peptidique de graines de légumineuse (Pisum sativum) commercialisé par la société LSN sous la dénomination commerciale Parelastyl^{®} ; les héparinoïdes ; et les pseudodipeptides tels que l'acide {2-[acétyt-(3-trifluorométhyl-phényl)-amino]-3-méthyl-butyrylamino} acétique.

La composition selon l'invention peut également contenir des agents conservateurs, tels que des esters de l'acide parahydroxybenzoïque, les agents stabilisants, des agents régulateur d'humidité, des agents émulsionnants, des filtres UVA et UVB , des antioxydants, tels que l'alpha-tocophérol, le butylhydroxyanisole ou le butylhydroxytoluène.

Selon un autre aspect de l'invention, celle-ci se rapporte à l'utilisation d'au moins un milieu de culture cellulaire ou d'au moins un extrait de milieu de culture, ledit milieu étant susceptible d'être obtenu par contact avec au moins une culture de cellules du tractus digestif et au moins des microorganismes probiotiques, tels que définis précédemment, pour la préparation d'un milieu de culture cellulaire et/ou tissulaire. Le milieu de culture conditionné selon l'invention ou ses extraits peuvent ainsi être utilisés comme adjuvant dans les systèmes de culture cellulaire, notamment pour améliorer les systèmes tridimensionnels et obtenir des conditions optimales de culture en système submergés ou émergés. En particulier, le milieu de culture conditionné et/ou ses extraits (et/ou les compositions les contenant) peuvent être utilisés pour la préparation d'un équivalent de peau in vitro.

En effet, le milieu de culture cellulaire conditionné ou ses extraits peuvent avantageusement être utilisés seuls ou en mélange avec un milieu de culture classique, pour cultiver des cellules in vitro, en particulier des cellules cutanées telles que les kératinocytes.
De tels systèmes de culture ont notamment été décrits par exemple dans les brevets EP285471, EP789074 ou EP857971 et comprennent une culture tri-dimensionnelle de kératinocytes, plus ou moins différenciés, sur un support. La culture peut en outre contenir d'autres types de cellules cutanés, telles que des mélanocytes, des cellules dendritiques ou des cellules de Langerhans, des fibroblastes, mais aussi des cellules nerveuses ou des cellules endothéliales.
Le support peut être constitué par un support inerte, en particulier un support poreux ou semi-perméable. Le support peut plus particulièrement être choisi parmi une matrice de collagène, comprenant éventuellement des fibroblastes et/ou des glycosaminoglycanes, un derme désépidermisé, un équivalent de derme, une membrane d'acide hyaluronique et/ou de collagène et/ou de fibronectine et/ou de fibrine, et un support inerte.

L'invention a également pour objet un procédé de préparation d'une composition, en particulier cosmétique ou dermatologique, comprenant une première phase au cours de laquelle on prépare un milieu de culture conditionné, éventuellement un extrait d'un tel milieu de culture conditionné.
Ce procédé comprend au moins les étapes suivantes:
a) culture de cellules dérivées de l'épithélium intestinal sur un support, notamment un support poreux dans un premier milieu nutritif pendant un temps suffisant pour obtenir leur différenciation
b) préparation d'un second milieu de culture cellulaire dans une enceinte tapissée de cellules de sang périphériques ou de leurs dérivées, notamment de leucocytes,
c) récupération de la culture de cellules différenciées sur le support, notamment poreux obtenue à l'issue de l'étape a) et transfert dans le second milieu de culture obtenu à l'issue de l'étape b)
d) mise en contact de ladite culture de cellules dérivées de l'épithélium intestinal différenciées dans le second milieu de culture, avec une culture de microorganismes comprenant au moins des probiotiques qui peuvent être par exemple de l'espèce *Lactobacillus* et/ou *Bifidobacterium,* pendant un temps suffisant pour qu'il y ait une interaction entre les cellules,
e) élimination des cultures cellulaires et récupération du second milieu de culture cellulaire, débarrassé des leucocytes, pour obtenir un milieu conditionné
f) incorporation du milieu conditionné ou d'un extrait de celui-ci dans une composition cosmétique ou dermatologique.

De préférence, le support utilisé à l'étape a) et suivant est un support poreux.
Par support poreux, on entend en particulier un insert dont la base comprend des pores.

Par exemple, la taille des pores pourra varier de 0,001 à 10 µm, de préférence supérieure ou égale à 0,3 µm.
A titre d'exemple non limitatif, la base de l'insert poreux convenant à l'invention, pourra ainsi comprendre une matrice poreuse de collagène, comprenant optionnellement des glycosaminoglycanes et/ou des fibroblastes, un gel ou une membrane d'acide hyaluronique et/ou de collagène et/ou de fibronectine et/ou de fibrine, une membrane semi-perméable de nitrocellulose, de nylon, de téflon, de polycarbonate ou de polyéthylène ou de polypropylène ou de polyethylène térephtalate (PET), une membrane inorganique Anopore^{®} semi-perméable, une membrane d'acétate de cellulose, une membrane semi-perméable Biopore-CM^{®}, une membrane semi-perméable de polyester et une membrane d'acide polyglycolique.

Le temps de contact à l'étape a) sera adapté par l'homme du métier mais sera généralement compris de quelques heures à quelques jours, notamment entre 1 jour et 35 jours, par exemple de 18 à 22 jours, de préférence d'environ 21 jours. Les cellules qui sont disposées initialement en couche unique forment ainsi à l'issue de l'étape a) un système à plusieurs couches de cellules différenciées, tridimensionnel. Avantageusement, le milieu de culture sera renouvelé régulièrement, par exemple tous les 2 jours, pour obtenir une différenciation optimale des cellules dérivées de l'épithélium intestinal, telles que les Caco2.
A l'étape b), les cellules de sang périphérique ou dérivées, en particulier des leucocytes, forment un tapis cellulaire; c'est-à-dire que la surface de l'enceinte est recouverte de façon sensiblement homogène par des leucocytes; ceux-ci peuvent être en monocouche ou en multicouche.
Ces leucocytes seront notamment recueillis à partir de prélèvement sanguin après au moins une étape de séparation, notamment par centrifugation, puis élimination au moins partielle des monocytes. Ils sont ensuite remis en suspension dans un milieu nutritif compatible avec leur viabilité, notamment du milieu RPMI.
De manière générale, les milieux pourront être choisis par l'homme du métier selon ses connaissances générales, et notamment parmi les milieux cités précédemment.

A titre d'exemple d'enceinte convenant à la mise en oeuvre de l'invention, il peut être mentionné des puits de plaques de culture telles que des plaques de culture cellulaire de 6, 12, 24, 48 puits ou de 96 puits, usuellement utilisées en culture de cellules.

Avantageusement, la culture de cellules différenciées sera lavée avec le milieu convenant à la viabilité des leucocytes avant le transfert à l'étape c). Le contact entre les cellules différenciées et les probiotiques, en présence des leucocytes sera effectué pendant un temps permettant l'établissement d'interaction, c'est-à-dire d'échanges chimiques ou biologiques entre les différents types cellulaires.
Au sens de l'invention, on entend désigner par l'expression "échange chimique cellulaire", l'ensemble des signaux figurés par des molécules, libérées à partir d'une cellule, et susceptible d'affecter, à distance, l'activité d'une autre cellule, appartenant ou non au même type cellulaire. Une telle molécule peut être, par exemple et de manière non limitative, un peptide, une protéine, un lipide, un sucre, une hormone stéroïdienne, une catécholamine. Elle peut être libérée sous forme d'une sécrétion.

Il est entendu que cette interaction peut intervenir en l'absence de contact physique direct entre les différents types cellulaires. En particulier, l'agencement dans lequel d'une part les cellules dérivées de l'épithélium intestinal et d'autre part le tapis cellulaire de leucocytes, disposés ou non dans une unique enceinte, sont mis en relation l'un avec l'autre au moyen du milieu de culture dans lequel ils sont incubés, sans que des cellules de la culture de cellules intestinales puissent entrer, directement, en contact avec des cellules d'un autre tapis, par exemple par contact entre les corps cellulaires ou au moyen de prolongements cellulaires.
Avantageusement, les microorganismes probiotiques sont ajoutés au niveau apical sur la culture de cellules intestinales.
Le temps convenant à l'établissement de cette interaction sera de quelques heures à plusieurs jours, généralement d'au moins 6 heures, de préférence d'au moins 10, notamment supérieur ou égal à 16 heures, mais pourra être prolongé sans inconvénient. On laisse par exemple les probiotiques en contact avec les cellules intestinales dans l'enceinte comportant les leucocytes pendant 6 à 36 heures.

Le milieu de culture est ensuite récupéré en le séparant de l'ensemble des cellules, par exemple en le récoltant au niveau basolatéral: ce milieu conditionné a été influencé par les deux types cellulaires et leur interrelation.
Comme indiqué précédemment, le milieu de culture ainsi récupéré, aussi appelé "milieu conditionné", contient de l'IL-1 0, généralement à une concentration supérieure ou égale à 20 pg/ml de milieu, notamment de 50 à 200 pg/ml.

Il peut ensuite subir des étapes de concentration, extraction, fractionnement connues en soi de l'homme du métier, avant l'introduction à titre d'ingrédient, dans une composition, notamment cosmétique ou pharmaceutique ou dermocosmétique selon l'invention.

La composition selon l'invention est destinée en particulier au traitement thérapeutique ou prophylactique des peaux et /ou muqueuses saines et /ou malades pouvant présenter des désordres de la peau, des phanères et/ou des cheveux tels que notamment :
- une peau ou des cheveux secs
- une altération de la fonction barrière cutanée
- une peau ou des cheveux sensibles
- une peau ayant des altérations au niveau de sa matrice extracellulaire
- des désordres liés à des thérapeutiques avec des antibiotiques ou des antimycosiques,
- des désordres entraînés par des dysrégulations hormonales (peaux grasses) ou a des états pelliculaires

De plus la composition pourra prévenir ou traiter certaines altérations liés au vieillissement chronologique.

L'invention sera illustrée plus en détail dans les exemples qui suivent.

### Exemple 1: Préparation d'un milieu conditionné

Des cellules proche des entérocytes humains, CaCo2 (entre le passage 60 à 65) sont ensemencés à la densité de 2.5 10⁵ cellules/ml dans un insert de culture de 25mm (ayant des nucleopore de 0,4µm, Becton Dickinson, Basel, SUISSE). Ces inserts sont placés dans une boites de culture (Nunc) et cultivés pendant 18 à 22 jours à 37°C/10% CO2 dans du DMEM (contenant de la glutamine et une forte concentration de glucose (Amimed, Allschwill, SUISSE) supplémenté avec des acides aminés non essentiels (Gibco, BRL), 10 mg/ml gentamycine (GIBCO BRL), et 0.1% de penicilline/streptomycine (10 000IU/ml et 10 000UG/ml) (GIBCO). Le milieu de culture est changé tous les 2 jours jusqu'à ce que les cellules soient complètement différentiées (21 jours). Une mesure de la résistance électrique trans épithéliale est déterminée continuellement lorsque les cellules CaCo2 sont confluentes en monocouche en utilisant une électrode Multicell-ERS (voltmètre/ohmmètre).
Par ailleurs des leucocytes sanguins de volontaires sains sont isolées à partir de sang périphérique de donneurs sains non apparentés, par centrifugation sur Lymphoprep. Les suspensions cellulaires (10⁷ cellules/ml) sont déposées dans une boîte de pétri et une incubation de 1 h30 à 37°C permet l'adhésion des monocytes. Ainsi, les leucocytes, majoritairement des lymphocytes T, contenus dans la population de cellules non adhérentes sont purifiés en utilisant leur propriété à former des rosettes en présence de globules rouges de mouton. Ces derniers sont ensuite éliminés par un choc osmotique en présence de NH₄CI (8,7mg/I). Dans tous les cas, la viabilité de la suspension de leucocytes est supérieure à 95%.

Ces leucocytes sont alors dilués dans du RPMI 1640 contenant 20% de sérum humain AB décomplémenté (56°C, 30 minutes, Sigma, St Louis, Missouri, USA).

Le modèle de coculture Caco2/leucocytes peut alors être réalisé. Pour cela les inserts de culture cellulaire de CaCo2 sont lavés 2 fois dans du milieu RPMI 1640 et transférés dans une plaque de culture de 6 puits contenant du milieu RPMI, préalablement tapissés avec des leucocytes (2x10⁶ cellules/ml) fraîchement purifiés. Ainsi, le leucocytes sont au niveau basolatéral et les CaCo2 sont au niveau apical.

La stimulation par des probiotiques de la cocultures CaCo2/leucocytes (globules blancs du sang périphériques) se réalise suivant les condition décrites ci-après:
Ainsi, au niveau apical, sont ajoutés 1x10⁷ cfu/ml de probiotiques. Le milieu de culture seul est utilisé comme contrôle négatif. Après la stimulation pendant de 6 à 36h (37°C, 10% CO2), de la coculture, le milieu conditionné est récolté au niveau basolatéral.
On utilise en particulier comme probiotique Lactobacillus paracasei, notamment la souche déposée à la CNCM sous le n°CNCMI-2116

### Exemple 2: effet sur la fonction barrière

Des milieux conditionnés obtenus selon l'exemple 1 mais après 16h de stimulation par 10⁸ cfu de Lactobacillus paracasei ont été mis en contact avec des peaux reconstruites Episkin® pendant 6 jours lors de la phase encore proliférative de ce modèle. La fonction barrière cutanée de ce modèle Episkin® résultant a ensuite été étudiée.

### PROTOCOLE

Les kits Episkin ont été reçus à J6, puis cultivés pendant la phase proliférative jusqu'à J13 selon cinq conditions
*1. Condition classique d'Episkir® (condition A) :* Traités de J6 à J13 avec le milieu de différenciation d'Episkin^{®}
   Le milieu de différentiation Episkin est celui décrit par Roguet R, Cohen C, Dossou KG, Rougier A :Episkin, a reconstructed human epidermis for assessing in vitro the irritancy of topically applied compounds (Toxicol in vitro 1993: 8: 283-291)
*2. Contrôle négatif (condition B)* : Traités de J6 à J13 avec 30% de milieu de contrôle négatif de culture (milieu conditionné issu de 16h00 de culture CaCO2/PBMC)
*3. Contrôle positif (condition C) :* Traités de J6 à J13 avec 30% de milieu conditionné CaCO2/PBMC + probiotique *(Lactobacillus paracasei* CNCMI-2116+ Bifidobacterium Lactis NCC 2818 (encore désigné Bbl2 ATCC27536))
Ainsi, Les kits ont été réceptionnés à J14 sur milieu de culture. Le milieu de culture a alors été remplacé par du milieu d'essai (qui est équivalent dans sa constitution au milieu de différenciation, avec à la place de 10% de Serum de veau foetal (SVF) uniquement 5% de SVF) pendant 24 heures à l'étuve à 37°C.

Ce milieu d'essai a ensuite été remplacé par 1.5 ml de PBS+tween 80 0.25% (p/p) puis mis à stabiliser à 32°C pendant 1 heure avant application. Les kits EPISKIN^{®} ont été utilisés dans leur nacelle.

Les conditions A, B, C ont été étudiés sur 6 puits pour chaque lot EPISKIN^{®}.

La pénétration du composé 2-nitro-para-phénylène diamine (colorant) est étudiée après différents traitements

### Conditions expérimentales

Le test est réalisé comme suit :
i) Formulation : Mélange entre la formulation contenant le colorant à 5 mM (1 part) et d'une solution tampon pH 9,5 (1 part).
Concentration finale du colorant dans formulation: 1 mM de matière première

| | | **Fournisseur** | **Pour 100g** |
|---|---|---|---|
| *formulation 1* | Alkyl (C8/C10 50/50) polyglucoside (2) en solution aqueuse à 60% tamponnée | SEPIC: oramix CG110 | 12g |
| | Eau déminéralisée | | 28 g |
| | Ethanol absolu pur | Prolabo | 20 g |
| | Alcool benzylique pur | Fluka | 4 g |
| | Polyethylène glycol (8 OE) 400 | | 6 g |
| | Eau déminéralisée | | qsp 100 g |
| | | | |

| | | **Fournisseur** | **Pour 1000 ml** |
|---|---|---|---|
| | Chlorure d'amonium (NH4CI) | | 54g |
| *Préparation de tampon pH 9,5* | Amoniaque en solution à 20% | Prolabo | Qsp pH 9,5 (env 20ml) |
| | Eau déminéralisée | | qsp. 1000 ml en fiole jaugée |

ii) Etude de pénétration sur cellules de diffusion :
- Température d'application :32°C
- Dose appliquée : 250 µl cm⁻²
- Durée d'application : 4 h
- Liquide récepteur : PBS, 0.25 % Tween 80 (avec 2,5mM Na-Vitaminé)
iii) Le liquide récepteur est collecté puis analysé en duplicate par HPLC. La concentration est déterminée à l'aide d'une gamme d'étalonnage faite le jour même à partir d'une solution mère à 0,5 mM dans le PBS, 0.25 % Tween 80³.
iv) Le taux de pénétration est calculé en rapportant la concentration mesurée à celle de la dose appliquée en tenant compte des volumes, soit : 70µl de formulation et 600 µl de liquide récepteur.

### Méthode HPLC et chromatogramme

Le système HPLC utilisé est un système Alliance 2695 et un détecteur Waters PAD 996, utilisé selon les spécifications du fabricant.

Pour l'analyse, des aliquotes des solutions de gamme et des échantillons à doser ont été prélevés et distribués dans des vials équipés d'insert. La quantification des échantillons de colorant a été effectuée par le système d'acquisition et de traitement Millenium v.3.2.

La condition A (i.e. condition classique d'EPISKIN^{®}) utilise le milieu de différenciation d'EPISKIN^{®} comme milieu de culture, qui est optimal pour la différenciation du modèle. La condition lot B (i.e. contrôle négatif) utilise donc un milieu de culture diminué de 30% de ce milieu de différenciation. Aussi, la fonction barrière présentée par la condition B a donc été retrouvée plus faible que celle de la condition A.

Le tableau suivant donne les taux de pénétration du composé de référence pour les conditions A B et C des lot EPISKIN^{®} exploités (condition à J14)

| | | | |
|---|---|---|---|
| | | | 03-epis- |
| | | | 4/07/2003 |
| Condition A | **lot A** milieu classique | mean | **7,96** |
| | | SD | 0,52 |
| | | CV | 6,52 |
| Condition B | **lot B** (+30% milieu non stimulé) | mean | **9,78** |
| | | SD | 2,21 |
| | | CV | 22,59 |
| | **lot C** (+30% milieu stimulé avec | | |
| Condition C | probiotique L. paracasei + Bifidobacterium Lactis NCC 2818 ) | mean | **7,15** |
| | | SD | 0,25 |
| | | CV | 3,45 |

Les résultats montrent une amélioration significative de la fonction barrière par rapport au contrôle négatif..

Seule la condition C conduit à une amélioration significative de la fonction barrière par rapport au contrôle négatif. L'utilisation de milieu conditionné stimulé avec les probiotiques permet d'obtenir une fonction barrière équivalente à celle obtenue dans des conditions standards.

### CONCLUSIONS

Ainsi il est démontré que la condition supplémentée par 30% de milieux conditionnés stimulés par les probiotiques (condition C) conduit à une amélioration significative de la fonction barrière.

### Exemple 3: Restauration de la fonction barrière

Le but de cette étude était d'évaluer l'effet de probiotiques sur la restauration de la fonction barrière (FB) de la peau excisée fraîche isolée en cellule de diffusion.
On a étudié l'influence des milieux conditionnés issus de co-cultures stimulées par le probiotique L. paracasei, obtenus comme décrit dans l'exemple 1, introduits dans le liquide récepteur. Cela permet d'évaluer l'effet sur la restauration de la fonction barrière, altérée par le sodium lauryl sulfate (SLS). Pour cela, des fragments de peau humaine récupérés en post-opératoire immédiat ont été installés dans un système de cellule de Franz® afin de mesurer la perte insensible en eau (PIE) au cours du temps.

L'évaluation a été réalisée sur des prélèvements de plastie abdominale fraiche. A J0, les fragments cutanés ont été installés en cellule de Franz®, à 32°C. Les fragments de peau en cellule de Franz® ont une surface de 10.18 cm2.

Le milieu de culture adapté au maintien en survie a été ajouté dans le compartiment récepteur des cellules de Franz (5,5 ml) complété avec les milieux de culture conditionnés dans la proportion de 30% (Ce milieu est celui décrit par Boisnic S, Branchet-Gumila MC, Merial-Kieny C, Nocera T; Skin Pharmacol Physiol 2005: 18 : 201-208.
Les conditions à tester sont les suivantes:
CONDITION 2 CONTRÔLE sur lequel il y aura un traitement avec du SLS (en "simplicate") : Milieu conditionné non stimulé (appelé milieu 2), correspondant à un milieu de culture conditionné simple mis en contact pendant 16 h avec le modèle reconstruit CaCO 2/PBMC)
CONDITION 3 (en "duplicate") : Milieu conditionné stimulé (appelé milieu 3), correspondant à un milieu de culture conditionné issu de la stimulation par le probiotique L. paracasei d'un modèle in vitro représentatif du système intestinal (CaCo2/PBMC) (stimulation par le L. paracasei à 10⁸ cfu/ml mis en contact pendant 16h avec le modèle reconstruit CaCO 2/PBMC)
CONDITION 4 sur lequel il y aura un traitement avec du SLS (en "duplicate") : Milieu conditionné stimulé (appelé milieu 3), correspondant à un milieu de culture conditionné issus de la stimulation par le probiotique L. paracasei d'un modèle in vitro représentatif du système intestinal (CaCo2/PBMC) (stimulation par L. paracasei à 10⁸ cfu/ml mis en contact pendant 16 h de stimulation avec le modèle reconstruit CaCO 2/PBMC)
2 donneurs sont analysés après altération de la fonction barrière selon le protocole suivant :
   A J0, la peau est préparée et montée sur des cellules de FRANZ en présence du milieu de culture
   Puis, celles-ci sont laissées 1 h afin qu'elles se rééquilibrent notamment au niveau du pH et des échanges d'eau avant une première mesure de PIE
   3 h après le montage, les milieux conditionnés sont ajoutés en prétraitement pour la nuit dans les cellules de Franz dans le milieu culture

### AJ1 1

Après une nuit de contact avec les milieux conditionnés, première mesure de la PIE le matin vers 8h30
Puis application du SLS sous occlusion à 10% : pour obtenir une altération de la fonction barrière significative mais réversible de notre explant monté en cellule de Franz, il faut appliquer 20 µl par cm2 pendant 24 h sous occlusion partielle (grâce à une feuille de paraffine étirée sur le compartiment donneur)

### AJ2

Nettoyage du surplus de SLS avec 600 µl d'eau milliQ (3 lavages puis séchage de la peau en tamponnant avec un coton-tige)
Changement de milieu dans les cellules de FRANZ
mesure de PIE 1 heure après, puis toutes les 2 h (4 fois par jour)

### AJ3

A 8h 30 du matin , mesure de PIE puis changement de milieu

Et mesure de PIE toutes les 2 heures (4 fois par jour)

### A J4

Mesure de PIE le matin , 8h30 et arrêt de la manipulation.

La PIE a été mesurée en dupliquet par cellule de Franz analysée. La sonde a été placée à la surface des prélèvements de peau.

### Résultats

**1^{ère} peau**

| **J0** à T1h | 5,2 ± 1,7 | | |
|---|---|---|---|
| **J0** à T4h | Condition 2 = **milieu 2 ajoute** | Condition 3 = **milieu** Condition 4 = **2 ou 3 ajouté milieu 3 ajouté** | |
| **J1** à T20h après montage des cellules de Franz | ± 0,8 | 4,45 ± 1,8 | |
| **J1** + SLS | SLS appliqué 3h | | SLS appliqué 3h |
| **J1**à T1h* | 20,7 ± 0 | 5,5 ± 2,2 | 10,5 ± 1 |
| **J1** à T3h* | 16,25 ± 1,25 | 5,4 ± 1,9 | 9,5 ± 0,8 |
| **J2** à T19h* | 7,65 ±0,65 | 4,7 ± 3,1 | 5,55 ± 1 |
| **J2** à T2lh* | 7,95 ± 0,15 | 5,6 ± 2,6 | 7,95 ± 0,2 |
| **J2** à T23h* | 9,5± 1,2 | 5,1 ± 2,95 | 8 ± 0,2 |
| **J2** à T26h* | 7,6 ± 0,4 | 5,07 ± 2,8 | 6,65 ± 0,1 |
| **J2** à T29h* | 7,3 ± 0°1 | 4,8 ± 1,7 | 9,25 ± 0,75 |
| **J3** à T45h* | 7,55 ± 0,45 | 5,3 ± 2,3 | 6,45 ± 1,5 |

| | | | |
|---|---|---|---|
| *: temps décompté après traitement au SLS des cellules concernées. | | | |

Le milieu conditionné stimulé par L. paracasei à 10⁸ufc selon l'invention limite l'altération de la fonction barrière altérée par le SLS et permet une reconstruction plus rapide de cette fonction, et identique à la normale (retour aux valeurs de PIE retrouvée sur les fragments sur lesquels aucun traitement par SLS n'a été appliqué).

**2^{ème} peau:**

| **J0** à T1h | 6 ± 1,7 | | |
|---|---|---|---|
| **J0** à T4h | Condition 2 = **milieu 2 ajouté** | Condition 3 = **milieu** 1 Condition 4 = **3 ajouté milieu 3 ajouté** | |
| **J1** à T20h après montage des cellules de Franz | ± 1 | 5 ± 0,9 | |
| **J1** + SLS | SLS appliqué 3h | | SLS appliqué 3h |
| **J1**à T1h* | 22,85 ± 1,25 | 3 ± 0,3 | 12,65 ± 0,5 |
| **J2** à T19h* | 11,9 ± 2 | 3,35 ± 1 | 8 ± 1,6 |
| **J2** à T21h* | 11,75 ± 0,45 | 3,4 ± 0,46 | 5,35 ± 0,7 |
| **J2** à T23h* | 12,5 ± 0,5 | 3,25 ± 0,4 | 5,8 ± 0,2 |
| **J2** à T26h* | 14± 0,2 | 3,4 ± 0,5 | 9,9 ± 2,1 |
| **J3** à T45h* | 8± 0,1 | 3,7 ± 0,3 | 4 ± 0,6 |

| | | | |
|---|---|---|---|
| *: temps décompté après traitement au SLS des cellules concernées | | | |

Comme dans le cas précédent, le traitement par du milieu conditionné selon l'invention limite l'altération de la fonction barrière altérée avec du SLS et permet une reconstruction rapide de cette fonction barrière

### Exemple 4 : Compositions topiques à appliquer sur la peau ou le cuir chevelu: (Les ingrédients son indiqués sous leurs dénominations CTFA)

### Composition pour préparer la peau au soleil

| | |
|---|---|
| Milieu conditionné stimulé* | 2,5% |
| Conservateurs | 1,35% |
| Sodium citrate | 0,035% |
| PEG-40 | 1,25% |
| Pentaerythrityl tetraethylhexanoate | 4% |
| Glycerin | 7% |
| Sorbitan tristearate | 0,3% |
| Prunus armeniaca Kermel oil | 2% |
| Cetyl alcohol | 0,7% |
| Propylene glycol | 2% |
| Triethanolamine | 0,4% |
| Cyclohexasiloxane | 2% |
| Carbomer | 0,75% |
| Tocopherol | 1 % |
| Silica | 2% |
| Ascorbyl glucoside | 0,1% |
| Polycaprolactone -béta carotène | 5% |
| Eau qsp | 100% |

| | |
|---|---|
| * obtenu selon l'exemple 1 | |

### Composition contenant des filtres solaires

| | |
|---|---|
| **Milieu conditionné stimulé**** | 3,5% |
| Mélange d'alcool cetylstearylique et | |
| d'alcool cetylstearylique oxyéthylene (33OE) 80/20 | 7,0% |
| Mélange de mono et de distearate de glycerol | 2,0% |
| Alcool cetylique | 1,5% |
| Poly dimethylsiloxane | 1,5% |
| Huile de vaseline | 15,0% |
| Butyl methoxydibenzoylmethane | 3,0% |
| Octocrylene | 7,0% |
| Glycerine | 20,0% |
| Eau déminéralisée | qsp 100% |

| | |
|---|---|
| **obtenu selon l'exemple 1, mais avec L. johnsonii (CNCM N°1225) comme probiotique | |

### Composition pour traiter la chute des cheveux :

| | |
|---|---|
| **lotion capillaire Milieu conditionné stimulé***** | 1% |
| Propylène glycol | 23% |
| Ethanol | 55% |
| Aminexil | 1,5% |
| Eau | qsp100% |

| | |
|---|---|
| *** obtenu selon l'exemple 1 mais avec B. Longum (CNCM N °2170) comme probiotique | |

### Composition contenant des antioxydants pour protéger la peau des dommages engendrés par la pollution, la fumée de cigarette

| | |
|---|---|
| **Milieu conditionné stimulé****** | 2,0% |
| Hydroxypropylcellulose (Klucel H vendu par la société Hercules) | 1,0% |
| Antioxydant-vitamine E | 2,0% |
| Isopropanol | 40,0% |
| Conservateur | 0,30% |
| Eau | qsp 100% |

| | |
|---|---|
| *** obtenu selon l'exemple 1, en utilisant un mélange de L. paracasei et B. longum | |

| | |
|---|---|
| **Lotion corporelle pour les peaux sèches** | |
| Huile minérale | 8,0% |
| Isopropyle palmitate | 5,0% |
| Polyglycéryl-3-düsostéarate | 4,0% |
| Octyldodecanol | 4,0% |
| Carbomère | 0,3% |
| **Milieu conditionné stimulé*** | 2,0% |
| Sodium cocoyglutamate | 2,0% |
| Conservateur | 0,5% |
| Parfum | 0,5% |
| Eau | qsp 100% |

| | |
|---|---|
| *obtenu selon l'exemple 1 | |

| | |
|---|---|
| **Shampoing antipelliculaire** | |
| Sodium Lauryl Sulphate | 7,0% |
| Cocamidopropylbetain | 2,0% |
| Sodium Lauryl sulphosuccinate | 2,0% |
| Extrait de Milieu conditionné** | 4,0% |
| Chlorure de sodium | 1,0% |
| Conservateurs | 0,5% |
| Parfum | 0,5% |
| Eau | qsp 100% |

| | |
|---|---|
| **extrait lyophylisé obtenu selon l'exemple 1 mais avec B. Lactis (NCC2818) comme probiotique | |

| | |
|---|---|
| Crème Arachidyl behenyl alcohol/arachidylglusoside | 3,0% |
| Isohexadecane | 7,0% |
| Huile d'amande douce | 3,0% |
| Beurre de Karité | 2,0% |
| Glycérine | 5,0% |
| Milieu conditionné stimulé* | 3,0% |
| BHT | 0,05% |
| POB méthyle | 0,1 % |
| POB propyle | 0,05% |
| Eau | qsp 100% |

| | |
|---|---|
| * obtenu selon l'exemple 1 | |

| | |
|---|---|
| Crème Extrait de Milieu conditionné***** | 1,5% |
| Glyceryl stearate et PEG 100 stéarate | 5,0% |
| Isohexadecane | 8,0% |
| Beurre de Karité | 5,0% |
| Glycérine | 3,0% |
| Carbopol 981 0,2% | 0,2% |
| Lubragel | 5,0% |
| Phenoxyéthanol | 1,0% |
| Soude | qsp pH6 |
| BHT | 0,05% |
| Dc 1503 | 1,0% |
| Eau | qsp 100% |

| | |
|---|---|
| *****extrait lyophilisé obtenu selon l'exemple 1 mais avec B. Bifidum comme probiotique | |

## Revendications

1. Composition cosmétique ou dermatologique contenant un milieu de culture cellulaire conditionné ou un extrait de celui-ci, ledit milieu étant susceptible d'être obtenu par contact avec au moins une culture de cellules du tractus digestif et au moins des microorganismes probiotiques.

2. Composition selon la revendication 1, **caractérisée en ce que** ledit milieu peut être obtenu par contact avec en outre des cellules de sang périphérique.

3. Composition selon la revendication 2, **caractérisée en ce que** les cellules de sang périphérique sont des leucocytes.

4. Composition selon l'une quelconque des revendications précédentes, **caractérisé en ce que** la culture de cellules du tractus digestif est une culture tri-dimensionnelle.

5. Composition selon l'une quelconque des revendications précédentes, **caractérisée en ce que** les cellules digestives sont des cellules épithéliales de l'intestin.

6. Composition selon l'une quelconque des revendications précédentes, **caractérisée en ce qu'**au moins un microorganisme probiotique est choisi dans le groupe suivant: *Saccharomyces, Yarrowia, Kluyveromyces, Torulaspora, Schizosaccharomyces pombe, Debaromyces, Candida, Pichia, Aspergillus, Penicillium, Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus* et *Lactobacillus.*

7. Composition selon l'une quelconque des revendications précédentes, **caractérisée en ce qu'**au moins un microorganisme probiotique est choisi parmi les bactéries lactiques, les bifidobactéries et les levures Saccharomyces.

8. Composition selon l'une quelconque des revendications précédentes, **caractérisée en ce qu'**au moins un micorganisme est choisi parmi les espèces *Lactobacillus et Bifidobacterium.*

9. Utilisation cosmétique d'au moins un milieu de culture cellulaire susceptible d'être obtenu par contact avec au moins une culture de cellules du tractus digestif et au moins des microorganismes probiotiques, tels que définis dans l'une des revendications précédentes, ou d'un extrait dudit milieu de culture cellulaire comme agent pour améliorer l'homéostasie de la peau.

10. Utilisation d'au moins un milieu de culture cellulaire ou de l'un de ses extraits selon la revendication 9, **caractérisée en ce que** ledit milieu est obtenu par contact avec en outre des leucocytes.

11. Utilisation d'au moins un milieu de culture cellulaire ou de l'un de ses extraits selon l'une des revendications 9 ou 10, **caractérisée en ce que** le milieu ou son extrait est utilisé comme agent pour améliorer la fonction barrière de la peau ou du cuir chevelu.

12. Utilisation d'au moins un milieu de culture cellulaire ou de l'un de ses extraits selon l'une au moins des revendications 9 ou 10, **caractérisée en ce que** le milieu ou son extrait est utilisé comme agent pour améliorer l'hydratation de la peau ou du cuir chevelu.

13. Utilisation d'au moins un milieu de culture cellulaire ou de l'un de ses extraits selon l'une au moins des revendications 9 à 12, **caractérisée en ce que** le milieu ou son extrait est utilisé comme agent pour retarder, diminuer et/ou prévenir les signes du vieillissement cutané.

14. Procédé cosmétique pour améliorer l'homéostasie cutané et/ou la fonction barrière et /ou l'hydratation de la peau ou du cuir chevelu, **caractérisé en ce qu'**on applique sur la peau ou le cuir chevelu au moins un milieu de culture cellulaire ou au moins un extrait de milieu de culture, ledit milieu étant susceptible d'être obtenu par contact avec au moins une culture de cellules du tractus digestif et au moins des microorganismes probiotiques, tels que définis dans l'une des revendications 1 à 8.

15. Utilisation d'au moins un milieu de culture cellulaire ou au moins un extrait de milieu de culture, ledit milieu étant susceptible d'être obtenu par contact avec au moins une culture de cellules du tractus digestif et au moins des microorganismes probiotiques, tels que définis dans l'une des revendications 1 à 8, pour la préparation d'une composition destinée au traitement des peaux sensibles.

16. Utilisation d'au moins un milieu de culture cellulaire ou au moins un extrait de milieu de culture, ledit milieu étant susceptible d'être obtenu par contact avec au moins une culture de cellules du tractus digestif et au moins des microorganismes probiotiques, tels que définis dans l'une des revendications 1 à 8, pour la préparation d'une composition destinée au traitement et/ou à la prévention des signes du stress cutané.

17. Utilisation d'au moins un milieu de culture cellulaire ou d'au moins un extrait de milieu de culture, ledit milieu étant susceptible d'être obtenu par contact avec au moins une culture de cellules du tractus digestif et au moins des microorganismes probiotiques, tels que définis dans l'une des revendications 1 à 8, pour la préparation d'un équivalent de peau in vitro.

18. Procédé de préparation d'une composition cosmétique ou dermatologique comprenant au moins un milieu de culture cellulaire ou l'un de ses extraits, **caractérisé en ce qu'**il comprend les étapes suivantes:
a) culture de cellules dérivées de l'épithélium intestinal sur un support poreux dans un premier milieu nutritif pendant un temps suffisant pour obtenir leur différenciation
b) préparation d'un second milieu de culture cellulaire dans une enceinte tapissée de leucocytes,
c) récupération de la culture de cellules différenciées sur le support poreux obtenue à l'issue de l'étape a) et transfert dans le second milieu de culture obtenu à l'issue de l'étape b)
d) mise en contact de ladite culture de cellules dérivées de l'épithélium intestinal différenciées dans le second milieu de culture, avec une culture de microorganismes comprenant au moins des probiotiques de l'espèce *Lactobacillus,* pendant un temps suffisant pour qu'il y ait une interaction entre les cellules,
e) élimination des cultures cellulaires et récupération du second milieu de culture cellulaire, débarrassé des leucocytes, pour obtenir un milieu conditionné
f) incorporation du milieu conditionné ou d'un extrait de celui-ci dans une composition cosmétique ou dermatologique.
